(19) Europäisches Patentamt
European Patent Office
Office européen des brevets

(11) Publication number: 0 287 899
A2

# (12) EUROPEAN PATENT APPLICATION

(21) Application number: 88105528.9

(22) Date of filing: 07.04.88

(51) Int. Cl.⁴: C08G 63/18 , C08L 67/02 , A61L 17/00

(30) Priority: 23.04.87 US 41515

(43) Date of publication of application: 26.10.88 Bulletin 88/43

(84) Designated Contracting States: BE CH DE ES FR GB GR IT LI NL SE

(71) Applicant: AMERICAN CYANAMID COMPANY
1937 West Main Street P.O. Box 60
Stamford Connecticut 06904-0060(US)

(72) Inventor: Bradley, Douglas P.
9 Tommy's Lane
Brookfield Connecticut(US)

(74) Representative: Wächtershäuser, Günter, Dr.
Tal 29
D-8000 München 2(DE)

## (54) Surgical closure device.

(57) A surgical closure device comprising an annealed fiber is disclosed. The fiber is a polymer or blend of butylene terephthalate. The closure device can be a suture or ligature. An improved process for manufacturing the suture or ligature is also disclosed.

EP 0 287 899 A2

# SURGICAL CLOSURE DEVICE

## BACKGROUND OF THE INVENTION

This invention relates to a surgical closure device. The closure device comprises an annealed fiber. The fiber is a polymer or blend of butylene terephthalate.

This invention specifically relates to a surgical suture or ligature. The suture or ligature has an inherent viscosity of 0.7 dl/g measured at a concentration of 0.5 g/ml. in m-cresol.

This invention also relates to an improved process for manufacturing a homopolymeric suture or ligature of butylene terephthalate.

One advantage of this invention comprises an unexpected reduction in the number of sutures that break during knot rundown. This advantage is extremely important and may even be critical during certain surgical procedures. That is, the reliability of the suture is improved because the user (usually a surgeon) has a greater degree of assurance that the suture will not break during the tying and/or subsequent rundown of a knot.

Prior art attempts to improve the knotting characteristics of a polyester suture have been by coating the suture fiber with polytetrafluoroethylene. This invention is an improvement over the prior art attempts.

This invention anneals a polybutyleneterephthalate homopolymeric fiber. The homopolymer in the fiber has an inherent viscosity of greater than 0.7 dl/g measured at a concentration of 0.5 g/ml in m-cresol.

A surgical closure device comprising an annealed fiber has been invented. The fiber consists essentially of at least one homopolymer having the formula:

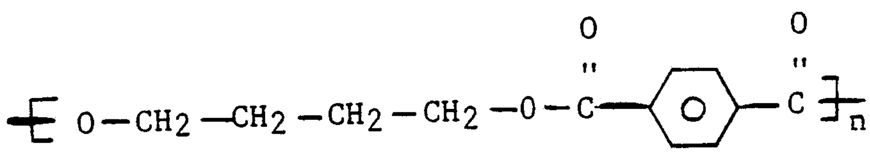

wherein n is the number of repeating units. The closure device has an inherent viscosity of more than 0.7 dl/g measured at a concentration of 0.5 g/ml in m-cresol. Throughout the disclosure of this invention, the term "an inherent viscosity of more than 0.7 dl/g "may be used without the clarifying term "measured at a concentration of 0.5 g/ml in m-cresol". If the latter term is not expressly stated, it is to be understood that it is implied.

In one embodiment, the closure device is a woven or knitted surgical fabric. The fabric is comprised of filaments of the annealed fiber described above. In a specific embodiment, the fabric is in a seamless tubular construction.

A surgical suture or ligature has also been invented. The suture or ligature comprises an annealed fiber. The fiber consists essentially of a homopolymer having the formula:

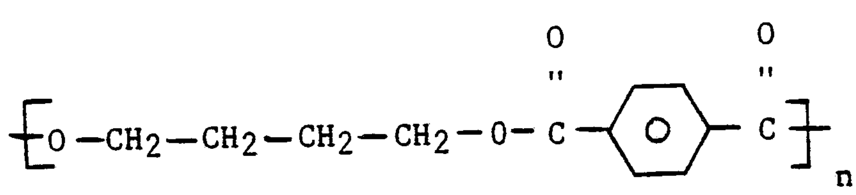

wherein n is the number of repeating units. The suture or ligature has an inherent viscosity of more than 0.7 dl/g measured at a concentration of 0.5 g/ml in m-cresol.

In one embodiment, the suture or ligature is in a sterile condition. In another embodiment, the suture has a needle attached to at least one end.

A surgical suture or ligature comprising a fiber manufactured from a composition selected from the group consisting of polybutylene (terephthalate-co-isophthalate); and a blend of two or more of the homopolymers, polybutyleneterephthalate, polybutylene isophthalate, and polyethyl ene terphthalate has also been invented. The suture or ligature has an inherent viscosity of more than 0.7 dl/g measured at a concentration of 0.5 g/ml in m-cresol.

In one embodiment, the suture or ligature is manufactured from polybutylene(terephthalate-co-isophthalate). In another embodiment, the suture or ligature is annealed. In yet another embodiment, the annealed suture or ligature is in a sterile condition. In a specific embodiment, the annealed suture has a needle attached to at least one end.

In all of the above embodiments, it is to be understood that n, the number of repeating units, can be calculated by any person skilled in the art without undue experimentation. For example, an inherent viscosity can be used to calculate a molecular weight. The number of repeating units can then be essentially obtained by dividing the total molecular weight obtained by the molecular weight of one repeating unit.

It is also to be understood that any person skilled in the art understands the upper limit of the described inherent viscosity without undue experimentation. That is, the fiber forming ranges of polymers (and specifically many if not most polyesters) are known from the prior art.

A process for manufacturing a suture or ligature has been invented. The prior art process comprises extruding and then sterilizing a fiber. The fiber consists essentially of at least the homopolymer having the formula:

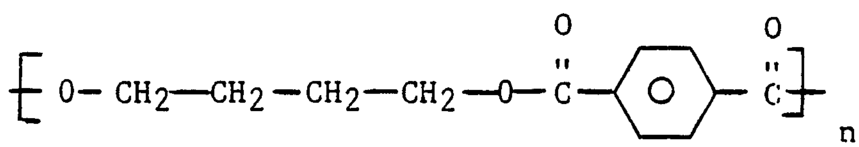

wherein n is the number of repeating units.

The improvement comprises between the extruding and sterilizing steps, maintaining the fiber at about a constant length and continuously annealing the fiber at a rate of about 10 to 15 meters per minute and at a temperature of about 180 to 210° Centigrade.

In one embodiment the annealing step is at a rate of up to about 20 meters per minute. In another embodiment, the temperature is at least about 200° Centigrade. Still another embodiment comprises batch annealing.

Still another embodiment is an annealed suture or ligature manufactured by the above disclosed process.

In a specific embodiment, the annealed suture or ligature has an inherent viscosity of more than 0.7 dl/g measured at a concentration of 0.5 g/ml in m-cresol. In a more specific embodiment, the annealed suture or ligature is in a sterile condition.

DESCRIPTION

Figure 1 is a schematic view of a test instrument for measuring knot rundown.

Referring to Figure 1, quantitative comparisons in knot tying properties are made using an Instron test instrument (hereafter abbreviated to Instron) model 1122 or 1130. The Instron is manufactured by Instron Corporation, Canton, MA, USA.

The Instron schematically shown in Figure 1 comprises two parts: the pulley assembly 1 which is rotably attached to a stationary base 2 of the Instron; and the rod assembly 3 which is connected to a movable crosshead 4 by two side frames 5 and a base frame 6.

To conduct a knot rundown test, a surgical suture strand 7 at least 27 inches in length is selected. The strand 7 is looped beneath the rod 3 and a single turn throw 8 is placed in the strand. Each end of the strand 7 is then threaded through the pulley arrangement 1 as shown. The strand ends are then fixed to the load cell 9 using flat faced pneumatic clamps.

Load cells are selected based on the size of the suture strand being tested: sizes 02 to 3-0 use a 2-100 kgf or a 5-50 kgf cell, and sizes 4-0 to 7-0 use a 100 to 2000 gf cell.

The throw 8 is manually maintained in approximately the position shown in Figure 1 while the rod assembly 3 is manually adjusted downward to apply a fixed pretension to the strand 7. Each suture size has been assigned a standard pretension.

The crosshead 4 is then raised at 1000 mm/min which forces the knot 8 to be rundown the strand 7 until it reaches the rod 3, at which time the crosshead is stopped.

A load trace is recorded on a standard Instron chart at a chart speed of 200 mm/min. Three tests are

conducted for each suture strand. If the strand breaks during the first or second test, the test is not repeated. A total of ten strands are tested for each suture strand size.

The data obtained comprises two parts: the number of strands that broke, sometimes expressed as a percentage; and the average force required to rundown a throw.

The polybutyleneterephthalate (PBT) suture has the chemical structure shown below:

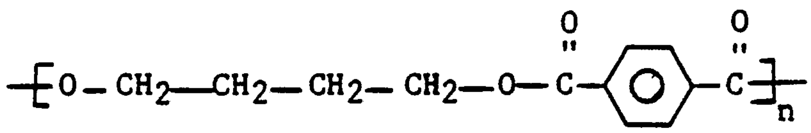

wherein n is the number of repeating units. Nonabsorbable monofilament PBT sutures, produced by a melt extrusion process, have approximately the following characteristics:

Straight Pull Breaking Strength (psi): greater than 40,000
Straight Pull Breaking Elongation (%): 25-40
Knot Pull Breaking Strength (psi): greater than 30,000
Flexural Modulus (psi): less than 1,000,000

Monofilament PBT sutures may be colored by the addition of a pigment. The pigment addition is typically accomplished by melt blending a pigment into the polymer at an initial concentration of about 5 to 10%. This initial concentration is then blended with nonpigmented polymer to achieve the desired concentration.

Prior to extrusion, the polymer or polymer/pigment charge is dried by exposure to heat (about 80-100°C) and vacuum (less than 50 mm Hg absolute).

After drying, the polymer charge is melted and extruded with a standard single-screw extruder at temperatures of about 215-250°C. Filaments are formed by pumping the molten polymer through a die and then rapidly cooling the strands in a water bath. The filaments are continuously pulled from this bath, heated to temperatures of about 140-200°C, and then oriented by stretching them between rolls. The rolls are rotating at successively higher speeds. The fiber is then wound onto spools at takeup speeds of 100-160 ft/min.

The spooled, oriented fiber is annealed or heat treated by continuously unwinding it and passing it over a series of heated rotating rolls at speeds of about 20-200 ft/min. and temperatures of 180-210°C, During this process, the fiber may be relaxed by overfeeding to the last roll before collecting again on spools.

A suture is manufactured from this fiber by attaching a needle to a strand of the fiber, inserting the needle and strand into a package, and then sterilizing the package, needle and strand. Sterilization may be accomplished by exposure to ethylene oxide or gamma radiation.

The preferred embodiments are more fully described by the following examples.

Example 1

Polybutyleneterephthalate homopolymer was extruded at temperatures of 225-250°C and at a rate of approximately 1.2 lb/hr. The single extruded strand was drawn 5.9x at temperatures of 180°C. Final takeup speed for the monofilament was 88 ft/min. The fiber was annealed by passing it over rotating rolls at speeds of 100 ft/min and temperatures of 210°C, with a relax of 2% allowed before takeup. Fiber properties were as follows:

| Diameter (mm) | Straight-Pull (psi) | Knot-Pull (psi) | Elongation (%) | Flexural Modulus (psi) |
|---|---|---|---|---|
| .568 | 84,800 | 49,700 | 28.5 | 399,000 |

Example 2

Polybutyleneterephthalate homopolymer was extruded by a process essentially similar to Example 1. The annealed fiber properties were as follows:

| Diameter (mm) | Straight-Pull (psi) | Knot-Pull (psi) | Elongation (%) | Flexural Modulus (psi) |
|---|---|---|---|---|
| .581 | 78,100 | 37,800 | 29.0 | 353,000 |

Example 3

Polybutyleneterephthalate homopolymer was extruded at temperatures of 225-250°C and at a rate of approximately 1.0 lb/hr. The single extruded strand was drawn 5.8x at temperatures of 160°C. Final takeup speed for the monofilaments was 163 ft/min. The fiber was annealed by passing it over rotating rolls at speeds of 100 ft/min. and temperatures of 210°C, with a relax of 4% allowed before take-up. Fiber properties were as follows:

| Diameter (mm) | Straight-Pull (psi) | Knot-Pull (psi) | Elongation (%) | Flexural Modulus (psi) |
|---|---|---|---|---|
| .391 | 80,300 | 41,900 | 30.8 | 532,000 |

The monofilament was attached with needles, packaged, and sterilized by exposure to gamma radiation. Suture properties were as follows:

| Diameter (mm) | Straight-Pull (psi) | Knot-Pull (psi) | Elongation (%) |
|---|---|---|---|
| .384 | 82,600 | 46,400 | 28.2 |

Example 4

Polybutyleneterephthalate homopolymer was extruded at temperatures of 225-250°C. and at a rate of approximately 0.8 lb/hr. The single extruded strand was drawn 5.6x at temperatures of 160°. Final takeup speed for the monofilaments was 185 ft/min. The fiber was annealed by passing it over rotating rolls at speeds of 100 ft/min. and temperatures of 210° C. with a relax of 6% allowed before takeup. Fiber properties were as follows:

| Diameter (mm) | Straight-Pull (psi) | Knot-Pull (psi) | Elongation (%) |
|---|---|---|---|
| .330 | 74,300 | 39,600 | 30.8 |

The monofilament was attached with needles, packaged, and sterilized by exposure to gamma radiation. Suture properties were as follows:

| Diameter (mm) | Straight-Pull (psi) | Knot-Pull (psi) | Elongation (%) |
|---|---|---|---|
| .338 | 71,300 | 37,100 | 30.8 |

Example 5

Polybutyleneterephthalate homopolymer was extruded by a process essentially similar to Example 4. The annealed fiber properties were as follows:

| Diameter (mm) | Straight-Pull (psi) | Knot-Pull (psi) | Elongation (%) | Flexural Modulus (psi) |
|---|---|---|---|---|
| .334 | 74,800 | 47,500 | 32.4 | 583,000 |

Example 6

Polybutyleneterephthalate homopolymer was extruded by a process essentially similar to Example 4. The annealed fiber properties were as follows:

| Diameter (mm) | Straight-Pull (psi) | Knot-Pull (psi) | Elongation (%) |
|---|---|---|---|
| .331 | 76,700 | 42,800 | 30.3 |

Example 7A

Polybutyleneterephthalate homopolymer was extruded at temperatures of 225-250°C. and at a rate of approximately 0.7 lb/hr. Four strands were extruded simultaneously and drawn 6x at temperatures of 160°C. Final takeup speed for the monofilaments was 150 ft/min. The fiber was annealed by passing it over rotating rolls at speeds of 100 ft/min. and temperatures of 210°C., with a relax of 6% allowed before takeup. Fiber properties were as follows:

| Diameter (mm) | Straight-Pull (psi) | Knot-Pull (psi) | Elongation (%) | Flexural Modulus (psi) |
|---|---|---|---|---|
| .247 | 85,400 | 50,700 | 29.2 | 542,000 |

The monofilament was attached with needles, packaged, and sterilized by exposure to gamma radiation. Suture properties were as follows:

| Diameter (mm) | Straight-Pull (psi) | Knot-Pull (psi) | Elongation (%) |
|---|---|---|---|
| .231 | 94,000 | 47,800 | 28.1 |

Example 7B

Polybutyleneterephthalate homopolymer was extruded by a process essentially identical to Example 7A. The annealed fiber properties were as follows:

| Diameter (mm) | Straight-Pull (psi) | Knot-Pull (psi) | Elongation (%) |
|---|---|---|---|
| .246 | 89,400 | 55,000 | 31.9 |

Example 8

Polybutyleneterephthalate homopolymer was extruded by a process essentially similar to Examples 7A and 7B. The annealed fiber properties were as follows:

| Diameter (mm) | Straight-Pull (psi) | Knot-Pull (psi) | Elongation (%) | Flexural Modulus (psi) |
|---|---|---|---|---|
| .234 | 90,600 | 54,200 | 31.0 | 582,000 |

Example 9

Polybutyleneterephthalate homopolymer was extruded at temperatures of 225-250°C. and at a rate of approximately 0.8x lb/hr. Four strands were extruded simultaneously and drawn 6x at temperatures of 160°C. Final takeup speed for the monofilaments was 150 ft/min. The fiber was annealed by passing it over rotating rolls at speeds of 100 ft/min. and temperatures of 210°C., with a relax of 9% allowed before takeup. Fiber properties were as follows:

| Diameter (mm) | Straight-Pull (psi) | Knot-Pull (psi) | Elongation (%) | Flexural Modulus (psi) |
|---|---|---|---|---|
| .199 | 83,600 | 56,200 | 29.3 | 630,000 |

The monofilament was attached with needles, packaged, and sterilized by exposure to gamma radiation. Suture properties were as follows:

| Diameter (mm) | Straight-Pull (psi) | Knot-Pull (psi) | Elongation (%) |
|---|---|---|---|
| .186 | 96,300 | 57,600 | 30.7 |

Example 10A

Polybutyleneterephthalate homopolymer was extruded at temperatures of 225-250°C. and at a rate of approximately 0.5x lb/hr. Eight strands were extruded simultaneously and drawn 6x at temperatures of 160°C. Final takeup speed for the monofilaments was 190 ft/min. The fiber was annealed by passing it over rotating rolls at speeds of 100 ft/min and temperatures of 210°C. with a relax of 9% allowed before takeup. Fiber properties were as follows:

| Diameter (mm) | Straight-Pull (psi) | Knot-Pull (psi) | Elongation (%) | Flexural Modulus (psi) |
|---|---|---|---|---|
| .086 | 111,600 | 88,600 | 28.2 | 691,000 |

The monofilament was attached with needles, packaged, and sterilized by exposure to gamma radiation. Suture properties were as follows:

| Diameter (mm) | Straight-Pull (psi) | Knot-Pull (psi) | Elongation (%) |
|---|---|---|---|
| .099 | 86,400 | 62,800 | 30.9 |

Example 10B

Polybutyleneterephthalate homopolymer was extruded by a process essentially identical to Example 10A. The annealed fiber properties were as follows:

| Diameter (mm) | Straight-Pull (psi) | Knot-Pull (psi) | Elongation (%) |
|---|---|---|---|
| .095 | 98,700 | 65,200 | 31.5 |

Example 11

Polybutyleneterephthalate homopolymer was extruded by a process essentially similar to Example 10. The annealed fiber properties were as follows:

| Diameter (mm) | Straight-Pull (psi) | Knot-Pull (psi) | Elongation (%) | Flexural Modulus (psi) |
|---|---|---|---|---|
| .095 | 85,200 | 68,200 | 32.6 | 681,000 |

TABLE 1

The following is a summary of the nonannealed physical data obtained from each of the above examples.

| Example No. | Size | Diameter (mm) | Straight-Pull Strength (kg) | Straight-Pull Strength (psi) | Knot-Pull Strength (kg) | Knot-Pull Strength (psi) | Breaking Elongation (%) | Knot-Rundown Breaks (breaks/10) | Knot-Rundown Force (g) | Knot-Run-down test Pretension (g) | Knot Run-down Pre-tension Ratio | Flexural Modulus (psi) |
|---|---|---|---|---|---|---|---|---|---|---|---|---|
| 1 | 02 | .568 | 14.28 | 80,100 | 7.07 | 39,700 | 20.90 | 0 | 2630 | 3000 | .88 | 391,000 |
| 2 | 02 | .561 | 15.31 | 88,000 | 6.05 | 34,800 | 20.55 | 0 | 2750 | 3000 | .92 | 363,000 |
| 3 | 1/0 | .374 | 6.89 | 89,200 | 3.44 | 44,500 | 19.00 | 1 | 1370 | 1500 | .91 | 667,000 |
| 4 | 2/0 | .320 | 4.40 | 77,800 | 2.66 | 47,000 | 19.40 | 0 | 815 | 800 | 1.02 | 496,000 |
| 5 | 2/0 | .324 | 4.66 | 80,300 | 2.28 | 39,300 | 20.00 | 0 | 785 | 800 | .98 | |
| 6 | 2/0 | .319 | 4.73 | 84,100 | 2.10 | 37,400 | 19.20 | 3 | 917 | 800 | 1.15 | 571,000 |
| 7 | 3/0 | .236 | 3.06 | 99,400 | 1.74 | 56,500 | 18.30 | 8 | 390 | 500 | .78 | |
| 8 | 3/0 | .225 | 2.86 | 102,300 | 1.53 | 54,700 | 17.90 | 6 | 493 | 500 | .99 | 535,000 |
| 9 | 4/0 | .184 | 1.88 | 100,500 | 1.04 | 55,600 | 17.20 | 0 | 210 | 250 | .84 | |
| 10 | 6/0 | .091 | .51 | 111,700 | .30 | 66,200 | 18.90 | 9 | 138 | 120 | 1.15 | 687,000 |
| 11 | 6/0 | .089 | .47 | 106,300 | .34 | 77,000 | 22.10 | 1 | 117 | 120 | .98 | 717,000 |

TABLE 2

The following is a summary of the annealed physical data obtained from each of the above examples.

| Example No. | Size | Diameter (mm) | Straight-Pull Strength (kg) | Straight-Pull Strength (psi) | Knot-Pull Strength (kg) | Knot-Pull Strength (psi) | Breaking Elongation (%) | Knot-Rundown Breaks (breaks/10) | Knot-Rundown Force (g) | Knot-Run-down test Pretension (g) | Knot Run-down Pre-tension Ratio | Flexural Modulus (psi) |
|---|---|---|---|---|---|---|---|---|---|---|---|---|
| 1 | 02 | .568 | 15.11 | 84,800 | 8.86 | 49,700 | 28.45 | 1 | 2590 | 3000 | .86 | 399,000 |
| 2 | 02 | .581 | 14.57 | 78,100 | 7.05 | 37,800 | 28.96 | 0 | 2493 | 3000 | .83 | 353,000 |
| 3 | 1/0 | .391 | 6.78 | 80,300 | 3.54 | 41,900 | 30.80 | 0 | 1197 | 1500 | .80 | 532,000 |
| 4 | 2/0 | .330 | 4.47 | 74,300 | 2.38 | 39,600 | 30.80 | 0 | 865 | 800 | 1.11 | |
| 5 | 2/0 | .334 | 4.61 | 74,800 | 2.93 | 47,500 | 32.40 | 0 | 865 | 800 | 1.08 | 583,000 |
| 6 | 2/0 | .331 | 4.64 | 76,700 | 2.59 | 42,800 | 30.30 | 0 | 856 | 800 | 1.07 | |
| 7* | 3/0 | .247 | 2.94 | 87,400 | 1.78 | 52,850 | 30.55 | 0 | 443 | 500 | .89 | 542,000 |
| 8 | 3/0 | .234 | 2.74 | 90,600 | 1.64 | 54,200 | 31.00 | 0 | 456 | 500 | .91 | 582,000 |
| 9 | 4/0 | .199 | 1.83 | 83,600 | 1.23 | 56,200 | 29.30 | 0 | 225 | 250 | .90 | 630,000 |
| 10* | 6/0 | .091 | .48 | 105,150 | .35 | 76,900 | 29.85 | 1 | 121 | 120 | 1.01 | 691,000 |
| 11 | 6/0 | .095 | .43 | 85,200 | .34 | 68,200 | 32.60 | 0 | 95 | 120 | .79 | 681,000 |

*Average of two lots except for flexural modulus which is from one lot.

TABLE 3

The following is a summary of the annealed and sterilized (by irradiation) physical data obtained from the above examples.

| Example No. | Size | Diameter (mm) | Straight-Pull Strength (kg) | Straight-Pull Strength (psi) | Knot-Pull Strength (kg) | Knot-Pull Strength (psi) | Breaking Elongation (%) | Knot-Rundown Breaks (breaks/10) | Knot-Rundown Force (g) | Knot-Run-down test Pretension (g) | Knot Run-down Pre-tension Ratio | Needle Pull Strength (kg) |
|---|---|---|---|---|---|---|---|---|---|---|---|---|
| 3 | 1/0 | .384 | 6.73 | 82,600 | 3.78 | 46,400 | 28.20 | 1 | 1107 | 1500 | .74 | 2.580 |
| 4* | 2/0 | .335 | 4.50 | 72,800 | 2.38 | 38,450 | 31.50 | 0 | 692 | 800 | .87 | 2.470 |
| 5* | 2/0 | .336 | 4.51 | 72,450 | 2.65 | 42,650 | 30.80 | 0 | 723 | 800 | .91 | 2.010 |
| 6 | 2/0 | .331 | 4.53 | 74,800 | 2.41 | 39,800 | 29.10 | 0 | 702 | 800 | .88 | 2.430 |
| 7* | 3/0 | .232 | 2.82 | 94,700 | 1.51 | 50,750 | 28.05 | 0 | 399 | 500 | .80 | 1.210** |
| 9+ | 4/0 | .186 | 1.79 | 93,500 | 1.11 | 58,100 | 29.70 | 1 | 310 | 250 | 1.24 | .830 |
| 10 | 6/0 | .099 | .47 | 86,400 | .34 | 62,800 | 30.90 | x | | | | .180 |

* Average of two lots.
\+ Average of three lots.
x No test conducted.
** One lot only.

Example 12

Three lots of polybutyleneterephthalate homopolymeric sutures, the suture fiber being the same or similar to one or more of the fibers described in Examples 1 to 11 above, were used in subcutaneous rat implants to compare the lot to lot variability of breaking strength after 30, 60, and 120 days.

The breaking strength variability from lot to lot was not significant. There was no loss of strength for any suture from 30 to 90 days.

Gross tissue reactions were unremarkable.

Example 13

Sizes 1/0, 2/0, 3/0 and 4/0 of polybutyleneterephthalate homopolymeric sutures, the suture fiber being the same or similar to one or more of the fibers described in the above Examples 3 to 9, respectively, were subcutaneously implanted in rats for 2, 7, 30, 60, 90, and 120 days.

At sacrifice the sutures were evaluated for gross tissue reaction and breaking strength. Gross tissue reaction was unremarkable at all time periods. At 120 days the percent strength retention ranged from 97% to 103%.

Example 14

The following values, based on the fibers prepared by the above examples, are calculated for several tenacity-elongation exchange relationships. The exchange relationships are defined in the prior art.

$$\text{Breaking Energy} = \text{Tenacity X Elongation;}$$

$$\text{Stress At Failure} = \text{Tenacity X}\left(1+ \frac{\text{Elongation}}{100}\right)\text{; and}$$

$$\text{Toughness ("Rosenthal Function")} = \text{Tenacity X Elongation}^{1/2}.$$

In these formulas, tenacity is measured in grams/denier and elongation is measured in percent.

As shown by contrasting the attached tables 4 and 5, annealing a PBT fiber increases its breaking energy and the Rosenthal Function but has no significant effect upon the true stress at failure. These results show that anneal ing can improve the PBT fiber toughness, therefore making it more difficult to break.

TABLE 4

Tenacity-Elongation Exchange Relationships Before Annealing

| Example No | Size | Diameter (mm) | Denier (g/9000 ) | S. P. (gram) | S. P. (g/d) | Elong. (%) | Rosenthal Function | Breaking Energy | Stress At Failure |
|---|---|---|---|---|---|---|---|---|---|
| 1 | 02 | 0.561 | 2892 | 15310 | 5.29 | 20.6 | 24.0 | 109 | 6.38 |
| 2 | 02 | 0.568 | 2965 | 14280 | 4.82 | 20.9 | 22.0 | 101 | 5.82 |
| 3 | 1/0 | 0.374 | 1285 | 6890 | 5.36 | 19.0 | 23.4 | 102 | 6.38 |
| 4 | 2/0 | 0.320 | 941 | 4400 | 4.68 | 19.4 | 20.6 | 91 | 5.58 |
| 5 | 2/0 | 0.324 | 965 | 4660 | 4.83 | 20.0 | 21.6 | 97 | 5.00 |
| 6 | 2/0 | 0.319 | 935 | 4730 | 5.06 | 19.2 | 22.2 | 97 | 6.03 |
| 7 | 3/0 | 0.236 | 512 | 3060 | 5.98 | 18.3 | 25.6 | 109 | 7.07 |
| 8 | 3/0 | 0.225 | 465 | 2860 | 6.15 | 17.9 | 26.0 | 110 | 7.25 |
| 9 | 4/0 | 0.184 | 311 | 1880 | 6.04 | 17.2 | 25.1 | 104 | 7.08 |
| 10 | 6/0 | 0.091 | 76 | 510 | 6.70 | 18.9 | 29.1 | 127 | 7.97 |
| 11 | 6/0 | 0.089 | 73 | 470 | 6.46 | 22.1 | 30.4 | 143 | 7.88 |
| | | | | Average | 5.58 | | 24.5 | 108 | 6.66 |

TABLE 5

Tenacity-Elongation Exchange Relationships

After Annealing (But Before Irradiation)

| Example No | Size | Diameter (mm) | Denier (g/9000m) | S. P. (gram) | S. P. (g/d) | Elong. (%) | Rosenthal Function | Breaking Energy | Stress At Failure |
|---|---|---|---|---|---|---|---|---|---|
| 1 | 02 | 0.581 | 3102 | 14570 | 4.70 | 29.0 | 25.3 | 136 | 6.06 |
| 2 | 02 | 0.568 | 2965 | 15110 | 5.10 | 28.5 | 27.2 | 145 | 6.55 |
| 3 | 1/0 | 0.391 | 1405 | 6780 | 4.83 | 30.8 | 26.8 | 149 | 6.31 |
| 4 | 2/0 | 0.330 | 1001 | 4470 | 4.47 | 30.8 | 24.8 | 138 | 5.84 |
| 5 | 2/0 | 0.334 | 1025 | 4610 | 4.50 | 32.4 | 25.6 | 146 | 5.95 |
| 6 | 2/0 | 0.331 | 1007 | 4640 | 4.61 | 30.3 | 25.4 | 140 | 6.01 |
| 7* | 3/0 | 0.247 | 559 | 2940 | 5.26 | 30.6 | 29.1 | 161 | 6.87 |
| 8 | 3/0 | 0.234 | 503 | 2740 | 5.45 | 31.0 | 30.3 | 169 | 7.13 |
| 9 | 4/0 | 0.199 | 364 | 1830 | 5.03 | 29.3 | 27.2 | 147 | 6.50 |
| 10* | 6/0 | 0.091 | 66 | 475 | 6.34 | 29.9 | 34.6 | 189 | 8.23 |
| 11 | 6/0 | 0.095 | 83 | 430 | 5.18 | 32.6 | 29.6 | 169 | 6.88 |
| | | | | Average | 5.16 | | 28.4 | 157 | 6.72 |

*Average of two lots.

## Claims

1. A surgical closure device comprising either an annealed fiber consisting essentially of at least one homopolymer having the formula:

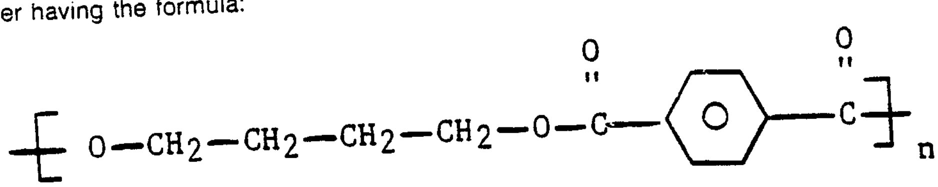

wherein n is the number of repeating units, or a fiber manufactured from a composition selected from the group consisting of polybutylene (terephthalate-co-isophthalate); and a blend of two or more of the homopolymers, polybutylene terephthalate, polybutylene isophthalate, and polyethylene terephthalate, the closure device having an inherent viscosity of more than 0.7 dl/g.

2. A surgical suture or ligature of claim 1 comprising an annealed fiber, the fiber consisting essentially of a homopolymer having the formula:

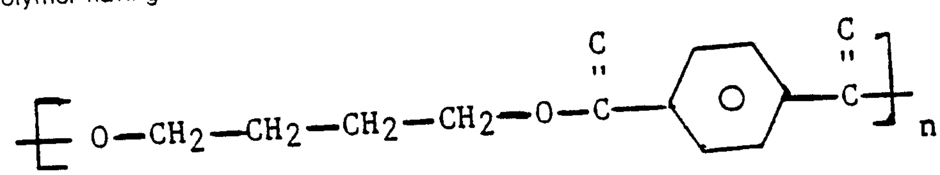

wherein n is the number of repeating units, the suture or ligature having an inherent viscosity of more than 0.7 dl/g.

3. A suture or ligature of claim 2 in a sterile condition.

4. A suture of claim 3 having a needle attached to at least one end.

5. A process for manufacturing a suture or ligature of claim 2 comprising extruding and then sterilizing a fiber by known prior art means, the fiber consisting essentially of at least the homopolymer having the formula:

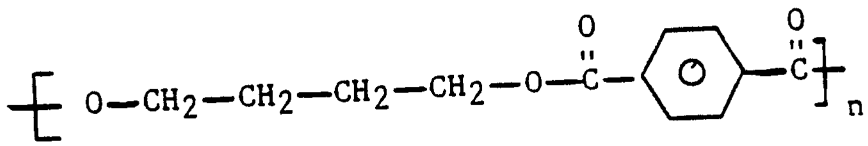

wherein n is the number of repeating units, the improvement comprising between the extruding and sterilizing steps, maintaining said fiber at about a constant length and annealing said fiber at a temperature of about 180 to 210° Centigrade.

6. A process of claim 5 comprising continuous annealing at a rate of about 10 to 50 meters per minute.

7. A process of claim 6 wherein the annealing step is at a rate of up to about 20 meters per minute.

8. A process of claim 7 at a temperature of at least about 200° Centigrade.

9. A process of claim 5 comprising batch anneal ing.

10. An annealed suture or ligature manufactured by the process of claim 5.

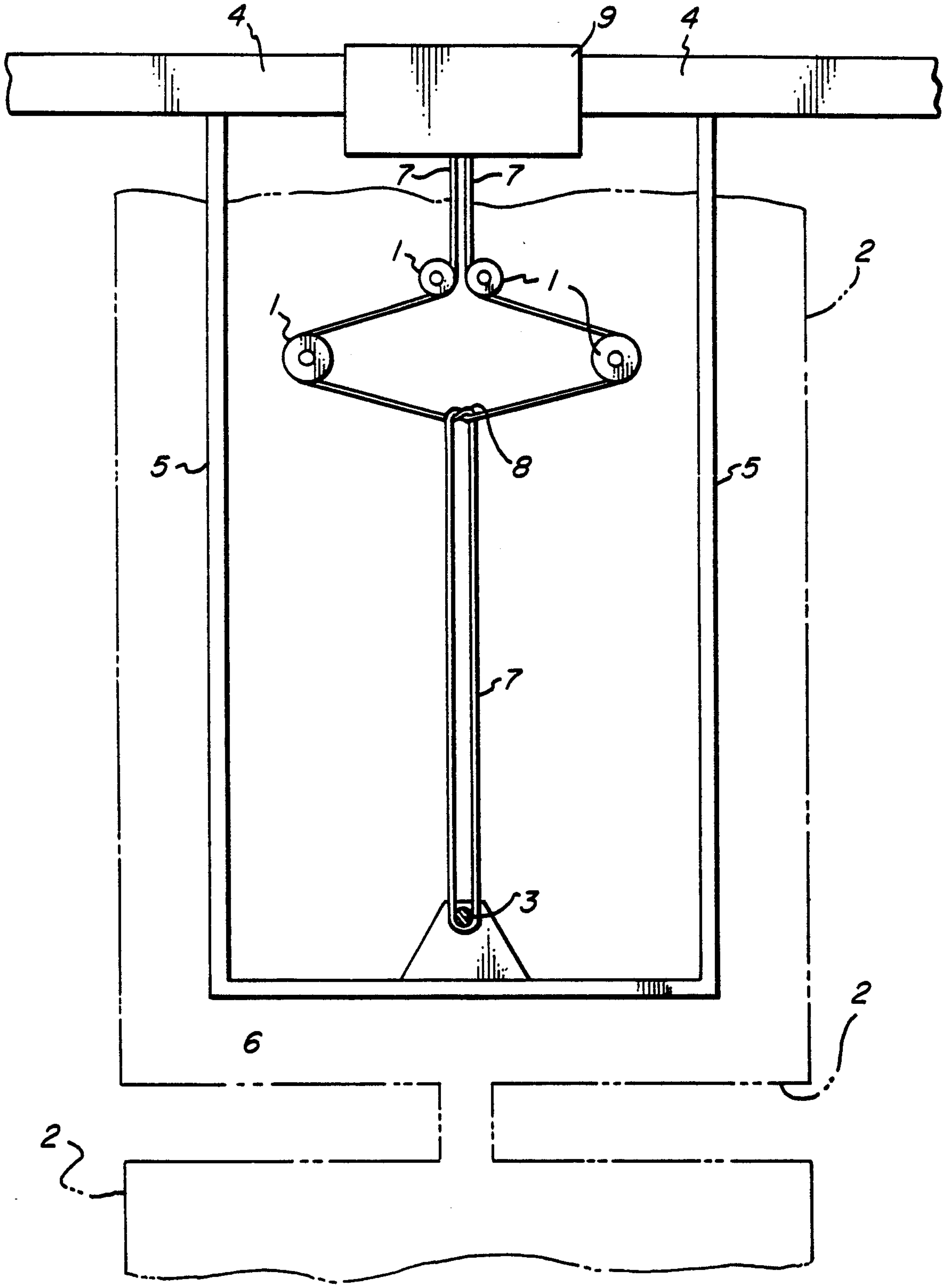
4
9
4
7
7
1
1
1
1
2
5
8
5
7
3
2
6
2